(19) Europäisches Patentamt
European Patent Office
Office européen des brevets

(11) Veröffentlichungsnummer: **0 135 477**
**B1**

(12) **EUROPÄISCHE PATENTSCHRIFT**

(45) Veröffentlichungstag der Patentschrift: **07.01.88**

(51) Int. Cl.⁴: **C 07 D 303/24,** C 08 G 59/32

(21) Anmeldenummer: **84810404.8**

(22) Anmeldetag: **17.08.84**

(54) **Polyglycidyläther.**

(30) Priorität: **23.08.83 CH 4577/83**
**18.04.84 CH 1947/84**

(43) Veröffentlichungstag der Anmeldung:
**27.03.85 Patentblatt 85/13**

(45) Bekanntmachung des Hinweises auf die Patenterteilung:
**07.01.88 Patentblatt 88/1**

(84) Benannte Vertragsstaaten:
**CH DE FR GB IT LI NL**

(56) Entgegenhaltungen:
**CH - A - 358 930**
**GB - A - 1 186 656**
**US - A - 3 558 535**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Renner, Alfred, Dr., Im Marcoup 2, CH-3280 Muntelier (CH)**
Erfinder: **Grütter, Peter, Dr., Tellistrasse 23/A, CH-5000 Aarau (CH)**
Erfinder: **Hügi, Rolf, Dr., Hombergstrasse 351, CH-4431 Ramlinsburg (CH)**

**EP 0 135 477 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft Polyglycidyläther, Verfahren zu ihrer Herstellung, härtbare Mischungen enthaltend diese Polyglycidyläther und die Verwendung der härtbaren Mischungen für den Oberflächenschutz und als Giessharze.

Polyglycidyläther cycloaliphatischer Alkohole sind bekannt. So ist zum Beispiel in H. Lee, K. Neville: Handbook of Epoxy Resins, (1967), McGraw Hill Co., New York, Seite 2–16 der Diglycidyläther von 2,2-Bis(p-hydroxycyclohexyl)-propan, bekannt als hydriertes Bisphenol A, genannt.

Es besteht jedoch weiterhin ein Bedarf an neuen hochreaktiven cycloaliphatischen Epoxidharzen mit guten technischen Eigenschaften, deren Ausgangsprodukte für die Synthese leicht zugänglich sind. Es wurden nun neue cycloaliphatische Epoxidharze gefunden, welche diesen Erfordernissen entsprechen.

Aus der US-A-3 558 535 sind Tetramethylolcyclohexanolderivate bekannt, die esterartig gebundene Epoxidgruppen tragen.

In der GB-A 1 186 656 wird ein Cyclohexan-1,1-dimethyloldiglycidyläther beschrieben.

Aus der CH-A 358 930 sind Kondensationsprodukte des Acetons mit Formaldehyd sowie deren Umsetzungsprodukte mit Epichlorhydrin bekannt. Diese Epoxidharze lassen sich in Kombination mit üblichen Härtern zur Herstellung von Giesslingen verwenden.

Gegenstand der vorliegenden Erfindung sind daher Polyglycidyläther der Formel (I)

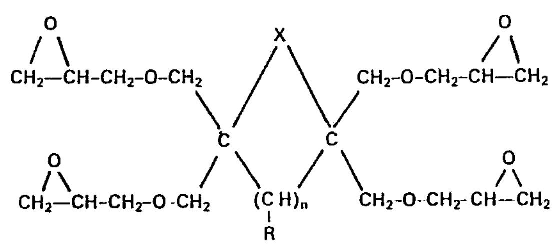

I

worin X eine Gruppe $-\overset{|}{C}=O$, $-\overset{|}{C}H-OH$ oder $-\overset{|}{C}H-O-CH_2-CH-CH_2$ bedeutet, R für Wasserstoff oder Methyl steht und n eine ganze Zahl von 2 bis 4 ist, wobei der Rest R innerhalb eines Moleküls verschiedene Bedeutungen annehmen kann.

Bevorzugt sind Tetraglycidyläther der Formel (I), worin X die Gruppe $-\overset{|}{C}H-OH$ oder $-\overset{|}{C}=O$ bedeutet.

Ebenfalls bevorzugt sind Polyglycidyläther der Formel (I), worin R Wasserstoff bedeutet.

n bedeutet vorzugsweise die Zahl 2 oder 3.

Besonders bevorzugt ist der Tetraglycidyläther der Formel I, worin X die Gruppe $-\overset{|}{C}H-OH$, R Wasserstoff und n die Zahl 3 bedeuten.

Die erfindungsgemässen Polyglycidyläther der Formel (I) kann man z.B. dadurch erhalten, dass man eine Verbindung der Formel (II)

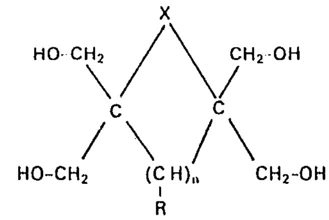

II

worin X, R und n die zuvor genannten Bedeutungen haben, mit einem Epihalohydrin in Gegenwart eines Katalysators zum entsprechenden Polyhalohydrinäther umsetzt und in Gegenwart eines Alkalimetallhydroxids dehydrohalogeniert.

Als Epihalohydrin kommt z.B. Epibromhydrin und insbesondere Epichlorhydrin in Frage.

Als Katalysatoren für die Umsetzung der Verbindung der Formel (II) mit Epihalohydrin eignen sich insbesondere Lewis-Säuren, wie z.B. $AlCl_3$, $SbCl_5$, $SnCl_4$, $FeCl_3$, $ZnCl_2$ und $BF_3$.

Die Umsetzung der Verbindungen der Formel (II) mit Epihalohydrin in Gegenwart einer Lewis-Säure wird vorzugsweise in Gegenwart eines inerten organischen Lösungsmittels durchgeführt. Als Lösungsmittel wird vorzugsweise 1,4-Dioxan verwendet.

Anstelle von reinem Dioxan kann man auch ein Lösungsmittelgemisch aus mindestens 30 Gewichtsprozent Dioxan und einem anderen organischen Lösungsmittel mit einem Siedepunkt von mindestens 70 °C, welches mit Epihalohydrin nicht reaktiv ist, verwenden. Geeignete organische Lösungsmittel, die in Kombination mit Dioxan, jedoch in einer 70 Gewichtsprozent des Lösungsmittelgemisches nicht übersteigenden Menge angewendet werden können, sind z.B. n-Heptan, n-Octan, 1,2-Dichloräthan, 1,2,2-Trichloräthylen, Benzol, Toluol, Xylol, Chlorbenzol, Methyläthylketon, Äthylacetat und n-Butylacetat.

Bevorzugt ist die Umsetzung in Gegenwart von $SnCl_4$ als Katalysator in 1,4-Dioxan.

Das Epihalohydrin wird in mindestens stöchiometrischen Mengen oder in einem leichten Überschuss, vorzugsweise 4–5 Mol, eingesetzt.

Als Katalysatoren für die Umsetzung mit Epihalohydrinen eignen sich ferner quaternäre Ammoniumsalze, vorzugsweise Salze von Ammoniumhalogeniden, wie z.B. Tetramethylammoniumchlorid, Tetraäthylammoniumbromid oder Benzyltrimethylammoniumbromid. Bevorzugtes Ammoniumhalogenid ist Tetramethylammoniumchlorid. Die quaternären Ammoniumsalze werden bevorzugt in Form wässriger Lösungen eingesetzt.

Die Umsetzung der Verbindungen der Formel (II) mit dem Epihalohydrin in Gegenwart eines Ammoniumsalzes wird vorzugsweise im entsprechenden Epihalohydrin als Lösungsmittel durchgeführt. Vorzugsweise verwendet man 2 bis 10

Mol Epihalohydrin pro Hydroxygruppe in der Formel (II).

Ferner kann die Umsetzung der Verbindungen der Formel (II) mit einem Epihalohydrin zu den entsprechenden Polyhalohydrinäthern in Gegenwart von Alkalimetallhydroxiden, wie z.B. Kalium- oder Natriumhydroxid, erfolgen. Die Alkalimetallhydroxide können beispielsweise als konzentrierte wässrige Lösungen zugesetzt werden.

Das Dehydrohalogenieren erfolgt zweckmässig mit Hilfe eines Alkalimetallhydroxids, vorzugsweise von NaOH, welches in äquivalenter Menge oder in schwachem Überschuss verwendet wird. Zweckmässigerweise verwendet man wasserfreies Natriumhydroxid oder dessen konzentrierte wässrige Lösung, vorzugsweise 50%ige Natronlauge. Alkalimetallhydroxide eignen sich demgemäss sowohl für die Umsetzung der Verbindungen der Formel (II) mit Epihalohydrinen als auch für die anschliessende Dehydrohalogenierung.

In manchen Fällen, z.B. wenn man quaternäre Ammoniumsalze für die Umsetzung einsetzt, wird die Glycidylierung vorteilhafterweise unter azeotroper Entfernung des Wassers in gleichzeitiger Gegenwart von Katalysator und Natronlauge durchgeführt. Die Natronlauge wird während der azeotropen Entwässerung unter vermindertem Druck zugesetzt. Wasser, das als Lösungsmittel für das NaOH dient, und solches, das bei der Reaktion gebildet wird, wird auf diese Weise laufend aus dem Reaktionsgemisch entfernt.

Das bei der Reaktion gebildete Salz, wie z.B. Kochsalz, wird entweder ausgewaschen, abfiltriert oder abzentrifugiert. Überschüssiges Epihalohydrin wird gegebenenfalls im Vakuum abdestilliert.

Die als Ausgangsverbindungen verwendeten Verbindungen der Formel (II) können beispielsweise nach dem Verfahren, wie in US-A-2 462 031 beschrieben, hergestellt werden. Das Epihalohydrin ist im Handel erhältlich oder kann nach bekannten Methoden hergestellt werden.

Die erfindungsgemässen Polyglycidyläther eignen sich im Gemisch mit üblichen Härtungsmitteln und gegebenenfalls mit Epoxidharzen auf der Basis von Bisphenol-A z.B. als Lack-, Giess-, Tauch- oder Imprägnierharzkomponente. Ein weiterer Gegenstand vorliegender Erfindung sind daher härtbare Mischungen, welche einen Polyglycidyläther der Formel (I) und ein Härtungsmittel für Epoxidharze enthalten.

Die erfindungsgemässen Polyglycidyläther sind im allgemeinen sehr hellfarbige Harze und ergeben nach der Härtung ausgezeichnete licht- und wetterechte Beschichtungen, mit zum Beispiel hoher Glanzbeständigkeit.

Übliche Härtungsmittel sind z.B. Carbonsäureanhydride, Verbindungen mit mehreren beweglichen Wasserstoffatomen, wie Polyamine, Polyamidoamine, Polythiole und mehrbasische Säuren. Gegebenenfalls unter Wärmeeinwirkung erfolgt die Überführung in den unlöslichen und unschmelzbaren Zustand. Auch anionische Polymerisationskatalysatoren, z.B. $BF_3$ und seine Komplexe, oder kationische, z.B. tertiäre Amine und Imidazole, eignen sich zum Herbeiführen und zur Beschleunigung der Härtung.

Gegenüber den meisten der genannten Härtungsmittel erweisen sich die erfindungsgemässen Verbindungen als reaktionsfähiger als bekannte Glycidyläther mehrwertiger Phenole, wie z.B. Resorcin, gegebenenfalls hydriertes Bisphenol A und Novolake. Ihre hohe Reaktivität verbunden mit guter Flexibilität der damit erhältlichen gehärteten Produkte machen sie besonders für Lackharze, Giessharze, Tauch- und Imprägnierharze geeignet. Infolge ihrer hohen Funktionalität kann nach der Härtung eine hohe Vernetzungsdichte erreicht werden. Beispielsweise kann man mit aliphatischen oder cycloaliphatischen Polyaminen Beschichtungen und Überzüge mit ausgezeichneter Chemikalienbeständigkeit erzeugen.

Den erfindungsgemässen härtbaren Epoxidharzmischungen kann man übliche Zusätze beimischen, wie aktive Verdünner zur Herabsetzung der Viskosität, Streck-, Füll- und Verstärkungsmittel, wie auch Pigmente, Farbstoffe, Weichmacher, Verlaufmittel, Thioxotropiermittel und flammhemmende Stoffe.

Speziell für die Anwendung auf dem Lackgebiet können die neuen Polyglycidyläther ferner in bekannter Weise mit Carbonsäuren, wie insbesondere höheren ungesättigten Fettsäuren partiell oder vollständig verestert werden. Es ist ferner möglich, solchen Lackharzformulierungen andere härtbare Kunstharze, zum Beispiel Phenoplaste oder Aminoplaste, zuzusetzen.

Die Herstellung der härtbaren Mischungen kann in üblicher Weise mit Hilfe bekannter Mischaggregate (Rührer, Kneter oder Walzen) etc., erfolgen.

Die härtbaren Epoxidharzmischungen finden ihren Einsatz vor allem auf den Gebieten des Oberflächenschutzes, der Elektrotechnik, der Laminierverfahren und im Bauwesen. Sie können in jeweils dem speziellen Anwendungszweck angepasster Formulierung, im ungefüllten oder gefüllten Zustand, gegebenenfalls in Form von Lösungen oder Emulsionen, als Anstrichmittel, Lacke, als (Wirbel-) Sinterpulver, Pressmassen, Spritzgussformulierungen, Tauchharze, Giessharze, Imprägnierharze, Bindemittel und Klebstoffe, als Werkzeugharze, elektrische Isolierstoffe, Laminierharze, Dichtungs- und Spachtelmassen, Bodenbelagsmassen und Bindemittel für mineralische Aggregate, verwendet werden. Bevorzugt ist ihre Verwendung für den Oberflächenschutz und Giessharze.

In den nachfolgenden Beispielen bedeuten, wenn nichts anderes angegeben ist, Teile Gewichtsteile und Prozente Gewichtsprozente.

Herstellungsbeispiele

Beispiel 1: Tetraglycidyläther von 2,2,6,6-Tetramethylolcyclohexanol

Man suspendiert 225 Teile Tetramethylolcyclohexanol in 420 Teilen Dioxan, gibt 18 Teile Zinntetrachlorid zu und erwärmt auf 100 °C, wobei

eine homogene Lösung entsteht. Man tropft unter Rühren 564 Teile Epichlorhydrin so zu, dass die Lösung immer schwach siedet. Anschliessend wird die Lösung während 2 Stunden unter Rückfluss gekocht, das Dioxan wird abdestilliert und der Rückstand wird in 1080 Teilen Toluol gelöst. Sodann destilliert man bei einem verminderten Druck von ca. 0,15 bar über einen Wasserabscheider und tropft im Verlauf von 5 Stunden 396 Teile wässrige, 50%ige Natronlauge bei 55 °C zu, wobei Wasser ständig entfernt wird. Nach Ende der Laugenzugabe setzt man die azeotrope Destillation noch während 30 Minuten fort. Man kühlt die Lösung auf 25 °C, wäscht mit Wasser und sodann mit einer 5%igen $NaHSO_4$-Lösung. Man trocknet das Reaktionsgemisch über $Na_2SO_4$, destilliert das Toluol unter vermindertem Druck ab und klärt den Rückstand durch Filtration bei 80 °C.

| | |
|---|---|
| Ausbeute: | 440 Teile (98% der Theorie) |
| Viskosität bei 25 °C | 2,61 Pa · s |
| Epoxidgehalt: | 7,4 Äq/kg |
| $Cl_{total}$: | 6,44% |
| $Cl_{hydrolysierbar}$: | 0,047% |
| Farbzahl nach Gardener und Holt: | 1–2 |

Beispiel 2:
Tetraglycidyläther von 2,2,6,6-Tetramethylolcyclohexanol

110 Teile Tetramethylolcyclohexanol und 15 Teile einer 50%igen wässrigen Lösung von Tetramethylammoniumchlorid werden bei 100 °C in 1850 Teilen Epichlorhydrin gelöst. Man kühlt die Lösung auf 55 °C und setzt durch Erniedrigung des Druckes auf circa 0,15 bar eine Destillation von Epichlorhydrin über einen Wasserabscheider in Gang. Im Verlauf von 3 Stunden tropft man 220 Teile einer 50%igen wässrigen Natronlauge zu, wobei gleichzeitig Wasser durch Destillation ständig entfernt wird. Nach Ende der Wasserabscheidung setzt man die Destillation unter Rückführung von Epichlorhydrin während 1 Stunde fort, kühlt das Reaktionsgemisch auf 30 °C ab, entfernt das ausgeschiedene Kochsalz durch Filtration und wäscht letzteres mit Epichlorhydrin nach. Man wäscht die vereinigte Epichlorhydrinlösung mit 150 Volumenteilen einer 10%igen wässrigen $NaH_2PO_4$-Lösung, wäscht mit Wasser nach und trocknet über $Na_2SO_4$. Man destilliert das Epichlorhydrin am Rotationsverdampfer ab und trocknet bei 140 °C und 0,02 bar. Man klärt den Rückstand durch Filtration über Cellite.

| | |
|---|---|
| Ausbeute: | 192 Teile (77% der Theorie) |
| Viskosität bei 25 °C: | 5,9 Pa · s |
| Epoxidgehalt: | 7,3 Äq/kg |
| $Cl_{total}$: | 0,75% |
| $Cl_{hydrolysierbar}$: | 0,085% |
| Farbzahl nach Gardener und Holt: | 3 |

Beispiel 3:
Tetraglycidyläther von 2,2,6,6-Tetramethylolcyclohexanon

Nach dem Verfahren von Beispiel 2 werden 110 Teile 2,2,6,6-Tetramethylolcyclohexanon in Gegenwart von 12 Teilen 50%igem, wässrigem Tetramethylammoniumchlorid mit 1100 Teilen Epichlorhydrin und 176 Teilen 50%iger wässriger Natronlauge umgesetzt. Man erhält ein gelbes Öl.

| | |
|---|---|
| Ausbeute: | 146,5 Teile (66% der Theorie) |
| Epoxidgehalt: | 6,57 Äq/kg |
| $Cl_{total}$: | 1,27% |
| $Cl_{hydrolysierbar}$: | 0,15% |

Beispiel 4:
Tetraglycidyläther von 2,2,5,5-Tetramethylolcyclopentanon

Man löst 102 Teile Tetramethylolcyclopentanon in 500 Teilen Dioxan bei 100 °C. Dazu gibt man unter Rühren und Rückflusskühlung 10 Teile $SnCl_4$ und 185 Teile Epichlorhydrin. Letzteres wird so zugetropft, dass die Dioxanlösung ohne äussere Erwärmung ständig siedet. Das Reaktionsgemisch wird weitere 2 Stunden unter Rückfluss gekocht. Man destilliert das Dioxan ab und löst den Rückstand in 750 Volumenteilen Toluol. Unter azeotroper Wasserabscheidung bei 55 °C und 0,15 bar fügt man 176 Teile 50%ige wässrige Natronlauge zu und setzt die Destillation noch 30 Minuten nach Beendigung der Laugenzugabe fort. Das entstandene Produkt wird vom Kochsalz durch Auswaschen mit Wasser befreit, sein pH wird mit 5%iger, wässriger $NaHSO_4$ auf 5–6 eingestellt, und schliesslich wird über $Na_2SO_4$ getrocknet.

Das Toluol wird unter vermindertem Druck abdestilliert. Man erhält ein rotes Harz.

| | |
|---|---|
| Ausbeute: | 136,5 Teile (64% der Theorie) |
| Epoxidgehalt: | 5,35 Äq/kg |

Beispiel 5:

Tetraglycidyläther von 4-Methyl-2,2,6,6-tetramethylolcyclohexanol

Nach dem Verfahren von Beispiel 2 werden 80 Teile 4-Methyl-2,2,6,6-tetramethylolcyclohexanol in Gegenwart von 11 Teilen 50%igem, wässrigem Tetramethylammoniumchlorid mit 1375 Teilen Epichlorhydrin und 163,6 Teilen 50%iger wässriger Natronlauge umgesetzt. Man erhält ein gelbliches Öl.

| | |
|---|---|
| Ausbeute: | 138,1 Teile (81% der Theorie) |
| Viskosität bei 25 °C: | 3,4 Pa · s |
| Epoxidgehalt: | 7,0 Äq./kg |
| $Cl_{total}$: | 4,2% |
| $Cl_{hydrolysierbar}$: | 0,57% |

Anwendungsbeispiele
Beispiel A

100 Gewichtsteile des Tetraglycidyläthers, hergestellt gemäss Beispiel 1, werden mit 46 Teilen eines Härters gemischt. Der Härter ist ein Polyaminaddukt-Härter und setzt sich aus 28 Teilen Bisphenol-A-Diglycidyläther, 57,7 Teile Trimethylhexamethylendiamin und 14,3 Teilen Phenol zusammen. Die Mischung hat eine Gebrauchsdauer von 10–15 Minuten bei einer Umgebungstemperatur von 20 °C und 65% relativer Feuchtigkeit. Ein 200 µ dicker Film härtet unter diesen Bedingungen innerhalb 3 Stunden durch.

Nach 7 Tagen Lagerung bei Raumtemperatur erhält man einen Film, der beständig ist gegen Aceton, Methyläthylketon, Essigsäureäthylester, Toluol und Xylol. Man erhält folgende Werte:

Härte nach Persoz: 300 sec.
Tiefungswert (DIN 53156): 6 mm

Beispiel B

100 Teile Tetraglycidyläther gemäss Beispiel 2 und 95,5 Teile Hexahydrophthalsäureanhydrid werden gemischt, in Formen von 150×150×4 $mm^2$ gegossen und 12 Stunden bei 120 °C und 6 Stunden bei 140 °C gehärtet. Man erhält hellgelbe, zähharte, einwandfreie Platten, die zu Prüfstäben zerschnitten werden. Folgende mechanische Eigenschaften werden danach bestimmt:

| | |
|---|---|
| Biegefestigkeit (ISO 178): | 100 $N/mm^2$ |
| Randfaserdehnung (ISO 178): | 5,7% |
| Schlagbiegefestigkeit (VSM 77105): | 11,5 $kJ/m^2$ |
| Wärmestandfestigkeit nach Martens: | 154 °C |
| Kochwasseraufnahme (1 h bei 100 °C) | 0,32% |

Beispiel C

100 Teile des Tetraglycidyläthers, hergestellt gemäss Beispiel 1, werden mit 125 Teilen eines Härters gemischt. Der Härter ist ein im Handel erhältlicher modifizierter Polyamidoamin-Härter mit den folgenden charakteristischen Eigenschaften:

| | |
|---|---|
| Amingehalt: | 200–225 |
| Viskosität bei 25 °C: | 3000–6500 mPa · s |
| Feststoffgehalt: | 100% |
| Farbzahl nach Gardener und Holt: | etwa 12 |
| Flammpunkt: | > 200 °C |

Das Tetraglycidyläther-Härter-Gemisch wird bei 25 °C 7 Tage lang gehärtet und anschliessend auf kalt-gewalzte Stahlplatten appliziert. Die beschichteten Platten werden anschliessend während 1000 Stunden mit einem Xenonlichtbogen-Bewitterungsapparat belichtet und deren Glanzbeständigkeit bei 60° in bestimmten Zeitabständen gemessen (ASTM* D-523).

Die Ergebnisse sind in der Tabelle zusammengefasst.

Tabelle

| | Glanzbeständigkeit bei 60° nach Stunden der Bestrahlung | | | | | | | | | | |
|---|---|---|---|---|---|---|---|---|---|---|---|
| | 0 | 100 | 200 | 300 | 400 | 500 | 600 | 700 | 800 | 900 | 1000 |
| Überzug gemäss Beispiel C | 88 | 90 | 87 | 91 | 80 | 89 | 85 | 91 | 81 | 81 | 93 |

Aus der Tabelle ist ersichtlich, dass die Glanzwerte über einen Zeitraum von 1000 Stunden praktisch konstant bleiben.

*ASTM = American Society for Testing and Materials

**Patentansprüche**

1. Polyglycidyläther der Formel (I)

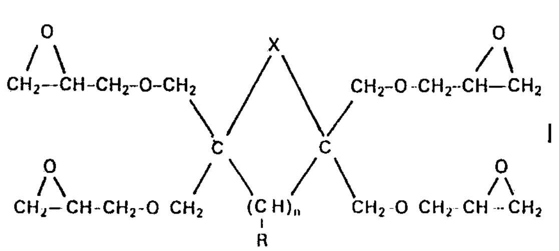

worin X eine Gruppe –C=O, –CH–OH oder –CH–O–$CH_2$–CH——$CH_2$ bedeutet, R für Wasserstoff oder Methyl steht und n eine ganze Zahl von 2 bis 4 ist, wobei der Rest R innerhalb eines Moleküls verschiedene Bedeutungen annehmen kann.

2. Tetraglycidyläther gemäss Anspruch 1, worin X die Gruppe –CH–OH oder –C=O bedeutet.

3. Polyglycidyläther gemäss Anspruch 1, worin R Wasserstoff bedeutet.

4. Polyglycidyläther gemäss Anspruch 1, worin n die Zahl 2 oder 3 ist.

5. Tetraglycidyläther gemäss Anspruch 1, worin X die Gruppe –CH–OH, R Wasserstoff und n die Zahl 3 bedeuten.

6. Verfahren zur Herstellung von Polyglycidyl-

äthern der Formel (I) gemäss Anspruch 1, dadurch gekennzeichnet, dass man eine Verbindung der Formel (II)

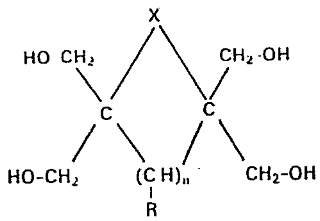

II

worin X, R und n die in Anspruch 1 genannten Bedeutungen haben, mit einem Epihalohydrin in Gegenwart eines Katalysators zum entsprechenden Polyhalohydrinäther umsetzt und in Gegenwart eines Alkalimetallhydroxids dehydrohalogeniert.

7. Verfahren gemäss Anspruch 6, dadurch gekennzeichnet, dass die Umsetzung in Gegenwart von $SnCl_4$ als Katalysator in 1,4-Dioxan erfolgt.

8. Verfahren gemäss Anspruch 6, dadurch gekennzeichnet, dass die Umsetzung in Gegenwart von Tetramethylammoniumchlorid als Katalysator erfolgt.

9. Verfahren gemäss Anspruch 6, dadurch gekennzeichnet, dass die Dehydrohalogenierung mit 50%iger Natronlauge erfolgt.

10. Härtbare Mischungen, enthaltend einen Polyglycidyläther gemäss Anspruch 1 und ein Härtungsmittel für Epoxidharze.

11. Verwendung der härtbaren Mischungen gemäss Anspruch 10 für den Oberflächenschutz und als Giessharze.

## Revendications

1. Ethers polyglycidyliques qui répondent à la formule I:

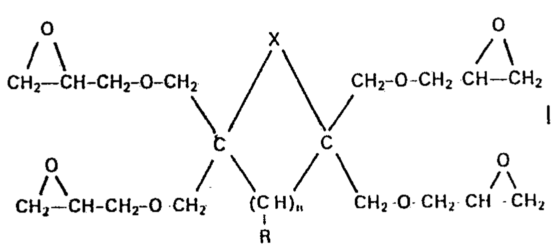

I

dans laquelle X représente un radical $-\overset{|}{C}=O$, $-\overset{|}{C}H-OH$ ou $-\overset{|}{C}H-O-CH_2-CH-CH_2$, R représente l'hydrogène ou un méthyle et n désigne un nombre entier de 2 à 4, le symbole R pouvant prendre des significations différentes à l'intérieur d'une même molécule.

2. Ethers tétraglycidyliques selon la revendication 1 dans lesquels X représente un radical $-\overset{|}{C}H-OH$ ou $-\overset{|}{C}=O$.

3. Ethers polyglycidyliques selon la revendication 1 dans lesquels R représente l'hydrogène.

4. Ethers polyglycidyliques selon la revendication 1 dans lesquels n est égal à 2 ou à 3.

5. Ether tétraglycidylique selon la revendication 1 dans lequel X représente le radical $-\overset{|}{C}H-OH$, R représente l'hydrogène et n est égal à 3.

6. Procédé de préparation d'éthers polyglycidyliques de formule I selon la revendication 1, procédé caractérisé en ce qu'on fait réagir un composé répondant à la formule II:

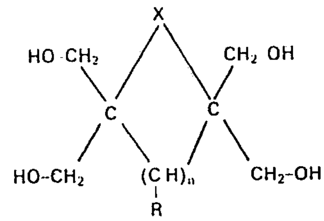

II

dans laquelle X, R et n ont les significations données à la revendication 1, avec une épihalogénhydrine, en présence d'un catalyseur, de manière à obtenir le poly-éther d'halogénhydrine correspondant, et on déshydrohalogène en présence d'un hydroxyde de métal alcalin.

7. Procédé selon la revendication 6 caractérisé en ce qu'on effectue la réaction dans du dioxanne-1,4 en présence de $SnCl_4$ comme catalyseur.

8. Procédé selon la revendication 6 caractérisé en ce qu'on effectue la réaction en présence de chlorure de tétraméthylammonium comme catalyseur.

9. Procédé selon la revendication 6 caractérisé en ce que la déshydrohalogénation est effectuée au moyen d'une solution à 50% d'hydroxyde de sodium.

10. Mélanges durcissables qui contiennent un éther polyglycidylique selon la revendication 1 et un durcisseur pour résines époxydiques.

11. Application des mélanges durcissables selon la revendication 10 pour la protection de surfaces et comme résines de coulée.

## Claims

1. Polyglycidyl ethers of the formula (I)

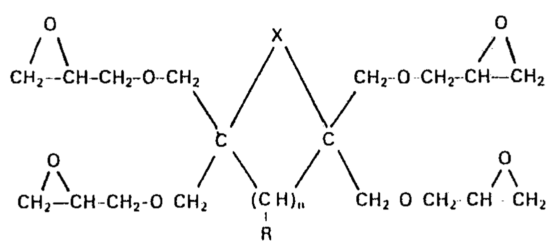

I

wherein X is a group $-\overset{|}{C}=O$, $-\overset{|}{C}H-OH$ or $-\overset{|}{C}H-O-CH_2-CH-CH_2$, R is hydrogen or methyl, and n is an integer from 2 to 4, where the radical R can have various meanings within one molecule.

2. Polyglycidyl ethers according to Claim 1, wherein X is the group $-\overset{|}{C}H-OH$ or $-\overset{|}{C}=O$.

3. Polyglycidyl ethers according to Claim 1, wherein R is hydrogen.

4. Polyglycidyl ethers according to Claim 1, wherein n ist the number 2 or 3.

5. Polyglycidyl ethers according to Claim 1, wherein X is the group $-\overset{|}{C}H-OH$, R is hydrogen, and n is the number 3.

6. A process for producing polyglycidyl ethers of the formula (I) according to Claim 1, which process comprises reacting a compound of the formula (II)

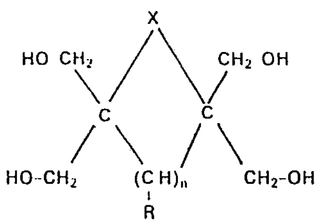

II

wherein X, R and n have the meanings defined in claim 1, with an epihalohydrin, in the presence of a catalyst, to give the corresponding polyhalohydrin ether, and dehydrohalogenating this in the presence of an alkali metal hydroxide.

7. A process according to Claim 6, wherein the reaction is performed in the presence of $SnCl_4$ as the catalyst in 1,4-dioxane.

8. A process according to Claim 6, wherein the reaction is performed in the presence of tetramethylammonium chloride as the catalyst.

9. A process according to Claim 6, wherein dehydrohalogenation is performed with a 50% sodium hydroxide solution.

10. Curable mixtures containing a polyglycidyl ether according to Claim 1, and a curing agent for epoxide resins.

11. Use of curable mixtures according to Claim 10, for surface protection and as casting resins.